# EUROPÄISCHE PATENTSCHRIFT

(11) **EP 1 806 157 B1**
(45) Veröffentlichungstag und Bekanntmachung des Hinweises auf die Patenterteilung: **06.11.2013**
(21) Anmeldenummer: 06025704.5
(22) Anmeldetag: 12.12.2006
(51) Int. Cl.: A61M 11/02, A61M 15/08, A61M 11/00

(54) **Aerosoltherapievorrichtung**
Aerosol therapy device
Appareil d'aerosoltherapie

(30) Priorität: 09.01.2006 DE 102006001113
(43) Veröffentlichungstag der Anmeldung: 11.07.2007
(73) Patentinhaber: PARI GmbH Spezialisten für effektive Inhalation, 82319 Starnberg (DE)
(72) Erfinder: Boehm, Andreas, 86934 Reichling (DE); Luber, Martin, 81379 München (DE); Rosenbeiger, Sven, 86920 Denklingen (DE)
(74) Vertreter: HOFFMANN EITLE

(56) Entgegenhaltungen:
- EP-A- 0 507 707
- WO-A-03/082393
- WO-A-2004/004814
- DE-B3- 10 239 321

## Beschreibung

Die Erfindung betrifft eine Aerosoltherapievorrichtung, bei der über ein Nasenstück ein in einer Verneblereinrichtung erzeugtes Aerosol in Form einer Aerosolhauptströmung den Nasenhöhlen eines Patienten zugeführt wird.

In diesem Zusammenhang ist aus "Eindringvermögen von Aerosolen in Nebenräume", H.Kauff, Archiv klin. exper. Ohren-, Nasen- und Kehlkopfheilk. 190, 95-108 (1968) bekannt, dass Druckschwankungen und Vibrationen der Anlass sein können, dass Aerosol in die Nasennebenhöhlen eindringt, die ansonsten von der Aerosolhauptströmung durch die Nasenhöhlen nicht aktiv durchströmt werden. Ein Anwendungsbeispiel dieser Erkenntnisse ist aus EP 0 507 707 A1 bekannt. Danach werden einer Aerosolströmung Druckschwankungen überlagert, die bewirken sollen, dass Aerosolpartikel/-tröpfchen der Aerosolhauptströmung die Ostien passieren und in die Nasennebenhöhlen gelangen. Auf diese Weise können auch die Nasennebenhöhlen, obwohl von der Aerosolhauptströmung nicht unmittelbar durchströmt, von einem in Aerosolform dargebotenen Medikament erreicht und therapiert werden. Wie auch bei anderen Aerosoltherapien wird angestrebt, dass Medikament in ausreichender Menge an den gewünschten Stellen zu deponieren, wozu im Fall der Nasennebenhöhlen eine ausreichende Menge des Aerosols der Aerosolhauptströmung die Ostien passieren und in die Nasennebenhöhlen eindringen muss.

Experimentelle Untersuchungen an verschiedenen Modellen der menschlichen Nase haben gezeigt, dass die Deposition in den Nasennebenhöhlen bei Anwendung der bekannten Aerosoltherapievorrichtungen geringer ist als erwartet und gewünscht. Auch die Öffnungsgröße der Ostien, die krankheitsbedingt oftmals sehr gering ist, hat einen starken Einfluss auf die Deposition.

WO 03/082393 A1 offenbart eine Vorrichtung zur Zufuhr einer Substanz in die Nasenhöhlen mit einer Versorgungseinrichtung mit einem Vernebler, der kontinuierlich ein Aerosolspray zuliefert, eine Gasversorgungseinheit, die durch ein Ausatmen betätigt wird und einen Gastrom in den Gasversorgungkanal liefert, der mit der Versorgungseinrichtung verbunden ist, ersten und zweiten Nasenstückeinheiten, die mit der Substanz-Versorgungseinrichtung verbunden sind, Strömungswiderstandseinheiten, die mit Eingängen der Ventile verbunden sind, und der ausströmenden Luft einen Strömungswiderstand entgegenstellen. Insbesondere ist die Gasversorgungseinheit in WO 03/082393 A1 so konfiguriert, dass eine Gasströmung mit einer alternieren Strömung bereitgestellt wird, um einen alternierender Druck in den Nasenhöhlen zu entwickeln.

Aus DE 102 39 321 B3 ist eine Aerosoltherapievorrichtung der oben beschriebenen Art bekannt, die einen Vernebler mit einem Aerosolgenerator, dem Druckluft für die Erzeugung einer Aerosolhauptströmung zugeführt wird, und mit einem Anschluss für die Zuführung von Druckschwankungen, die der Aerosolhauptströmung überlagert werden, und ein Nasenstück für die Zuführung des Aerosols in einen der beiden Nasenflügel eines Benutzers, das mit dem Vernebler verbunden ist, umfasst. Ferner ist eine Strömungswiderstandseinrichtung vorgesehen, mit der der Strömungswiderstand am anderen der beiden Nasenflügel des Benutzers genau definiert wird. Durch den Strömungswiderstand an der anderen Nasenöffnung führen die überlagerten Druckschwankungen in deutlich stärkerem Umfang dazu, dass Aerosol aus der Aerosolhauptströmung auch in die Nasennebenhöhlen gelangt und dort eine Deposition des Aerosols stattfindet.

Die in DE 102 39 321 B3 beschriebene Zuführung der Druckgasströmung und der Druckschwankungen erfordert aber eine besondere Gestaltung des Verneblers, so dass sich nicht jeder Vernebler für diese Anwendung eignet.

Die Erfindung verfolgt vor diesem Hintergrund das Ziel, geeignete Maßnahmen aufzuzeigen, durch die auch andere Vernebler im Rahmen einer Aerosoltherapie der eingangs geschilderten Art bei praktisch gleicher oder besserer Deposition des Aerosols in den Nasennebenhöhlen eingesetzt werden können, so dass auch mit anderen Verneblern eine therapeutisch sinnvolle und vorhersagbare Deposition in den nicht aktiv durchströmten Nasennebenhöhlen erzielbar wird.

Dieses Ziel wird erreicht durch eine Aerosoltherapievorrichtung mit den Merkmalen gemäß Patentanspruch 1. Vorteilhafte Ausgestaltungen ergeben sich aus den Unteransprüchen.

Eine Gerätekonfiguration gemäß der Erfindung zeichnet sich insbesondere dadurch aus, dass man damit gezielt Krankheiten des oberen und unteren Respirationstrakts behandeln kann, wenn diese eine nasale oder paranasale Ursache haben.

Ferner ist für eine Gerätekonfiguration gemäß der Erfindung kennzeichnend, dass flüssige Arzneiformulierungen damit besonders vorteilhaft in Form eines Aerosolnebels in die paranasalen Kavitäten deponiert werden können, um Krankheiten zu behandeln, die dort ihren Ursprung haben.

Außerdem ist eine Gerätekonfiguration gemäß der Erfindung auch dadurch gekennzeichnet, dass durch gezielte Deposition von Wirkstoffen in die Nase und paranasalen Kavitäten, Krankheiten wie chronische oder allergische Sinusitis, Entzündungen oder Infektionen oder andere Krankheits- oder Befindlichkeitszustände ("Stinkenase") topisch behandelbar sind, um dadurch unerwünschte Nebenwirkungen einer systemischen Arzneitherapie zu vermeiden.

Im Folgenden wird die Erfindung anhand von Ausführungsbeispielen unter Bezugnahme auf die Zeichnungen genauer erläutert. In den Zeichnungen zeigt:
- Figur 1: eine Ansicht einer ersten Verneblereinrichtung für den Einsatz in einer erfindungsgemäßen Aerosoltherapievorrichtung;
- Figur 2: eine erste Ansicht einer zweiten Verneblereinrichtung für den Einsatz in einer erfindungsgemäßen Aerosoltherapievorrichtung
- Figur 3: eine zweite Ansicht der zweiten Verneblereinrichtung für den Einsatz in einer erfindungsgemäße Aerosoltherapievorrichtung;
- Figur 4: eine Ansicht einer erfindungsgemäßen Strömungswiderstandseinrichtung;
- Figur 5: eine weitere Ansicht der erfindungsgemäßen Strömungswiderstandseinrichtung;
- Figur 6: eine Ansicht einer Einrichtung für die Erzeugung von Druckschwankungen;
- Figur 7: ein Diagramm zur Bestimmung wirksamer Durchmesser/Längen-Wertepaare für eine erfindungsgemäße Strömungswiderstandseinrichtung,
- Figur 8: eine Ansicht eines Nasenstücks mit verbundener Strömungswiderstandseinrichtung, und
- Figur 9: eine Ansicht eines erfindungsgemäßen Nasenstücks zur Aufbringung von Druckschwankungen.

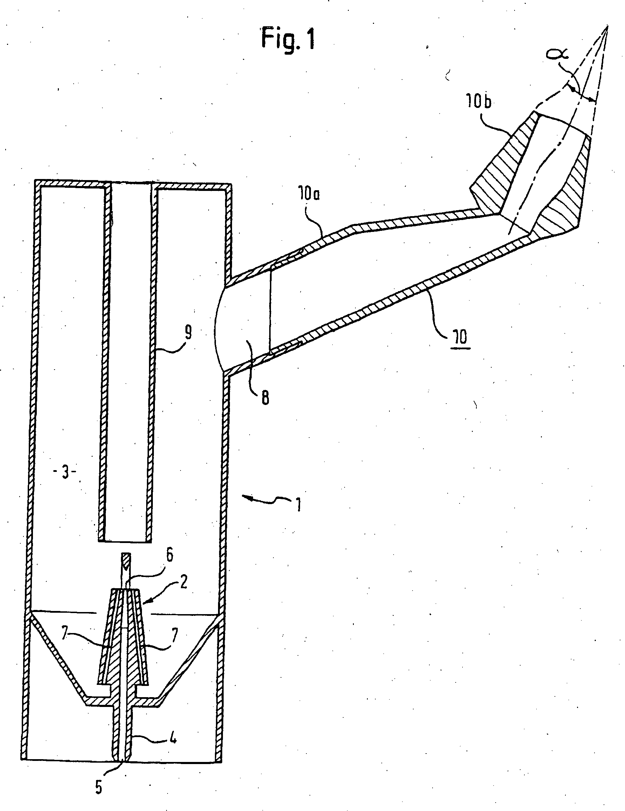

Figur 1 zeigt ein erstes Beispiel einer Verneblereinrichtung, die im Rahmen der Erfindung für eine eingangs beschriebene Aerosoltherapie verwendet werden kann. Die Verneblereinrichtung 1 umfasst einen Aerosolgenerator 2, der in einer Vernebelungskammer 3 angeordnet ist. Eine am Fuß des Aerosolgenerators bevorratete Flüssigkeit wird mit Hilfe des Aerosolgenerators 2 vernebelt, wenn dem Aerosolgenerator 2 über einen Anschluss 4, der an einem Ende (in Fig. 1 unten) des Aerosolgenerators angeordnet ist, Druckluft zugeführt wird. Die Druckluft durchströmt einen zentral in dem Aerosolgenerator angeordneten Druckluftkanal 5 und tritt am anderen Ende des Aerosolgenerators aus einer Düsenöffnung 6 aus. Über neben dem Druckluftkanal angeordnete Ansaugkanäle 7, die sich in dem Aerosolgenerator von der Ebene der Düsenöffnung bis zum Fuß des Aerosolgenerators erstrecken und zur dort bevorrateten Flüssigkeit hin öffnen, wird die Flüssigkeit angesaugt und im Bereich vor der Düsenöffnung 6 in die Vernebelungskammer 3 hinein vernebelt.

Bei auf die unteren Atemwege, den Bronchialtrakt und die Lunge ausgerichteten Aerosoltherapien atmet ein Patient das auf diese Weise erzeugte Aerosol ein, in dem er über ein Mundstück, das an einem Anschlussstutzen 8 der Verneblereinrichtung angebracht ist, beim Einatmen das Aerosol aus der Verneblereinrichtung entnimmt. Dabei strömt über einen Zuluftkamin 9 nach Bedarf Umgebungsluft in die Vernebelungskammer 3 nach, wenn das Aerosol während der Einatmungsphase aus der Vernebelungskammer 3 entnommen wird.

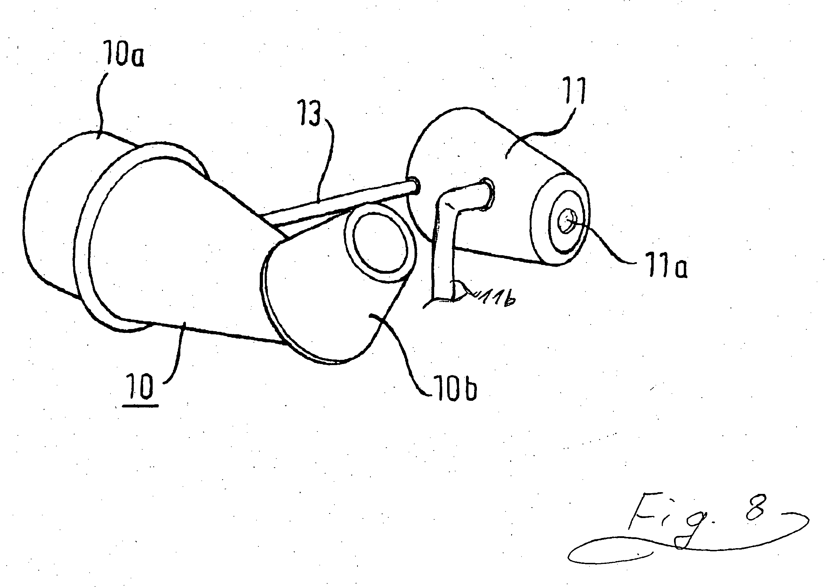

Bei auf den Nasenraum ausgerichteten Aerosoltherapien tritt an die Stelle des Mundstücks ein Nasenstück 10, das an einem Ende 10a für die Befestigung an dem Anschlussstutzen 8 der Verneblereinrichtung 1 ausgelegt ist, während das andere Ende 10b so gestaltet ist, dass es in eine Nasenöffnung der Nase eines Patienten eingeführt werden kann und diese dicht verschließt. Das Ende 10b ist vorzugsweise in Form eines Kegelstumpfes mit einem Öffnungswinkel a im Bereich von 10° bis 40° gestaltet. Dabei ist die Längsachse des Kegelstumpfes so gegenüber der Längsachse des Anschlussstutzens 8 geneigt, dass bei senkrechter Haltung des Verneblers ein einfaches und angenehmes Plazieren des Nasenstücks in der Nasenöffnung der Patienten gewährleistet ist. Bezüglich der vorangegangenen Beschreibung des Nasenstücks 10 wird außerdem auf die Figur 8 verwiesen.

Auf diese Weise wird der einen Nasenöffnung und damit der einen Nasenhöhle des Patienten das in der Verneblereinrichtung erzeugte Aerosol zugeführt. Die Druckluft, die für die Erzeugung des Aerosols der Verneblereinrichtung zugeführt wird, stellt in ausreichendem Maße eine Aerosolhauptströmung in bzw. durch die Nase des Patienten sicher. Die Aerosolhauptströmung verläuft von der einen Nasenöffnung durch die eine Nasenhöhle in die andere Nasenhöhle. Dieser Aerosolhauptströmung werden Druckschwankungen überlagert, wie weiter unten noch ausführlicher beschrieben wird. Ohne weitere Maßnahmen tritt die Aerosolhauptströmung aus der anderen Nasenöffnung der Nase des Patienten aus, wenn der Patient, wie bei Aerosoltherapien für den Nasenraum üblich, die Nasenhöhlen mit Hilfe des Gaumensegels zum Rachen und Mund hin verschließt.

Bei dem zuvor geschilderten ersten Beispiel einer für die Anwendung der Erfindung geeigneten Verneblereinrichtung wird aufgrund der Zuführung eines Druckgases für die Aerosolerzeugung eine Aerosolhauptströmung quasi systembedingt ebenfalls erzeugt. Zumindest kann der Druckgasstrom herangezogen werden, um die gewünschte Aerosolhauptströmung zu generieren. Andere Verneblereinrichtungen erzeugen aber ein Aerosol, ohne dass ein Druckgas zugeführt wird, beispielsweise mit Hilfe einer schwingenden Membran oder mit einem Ultraschallschwinger. In diesen Fällen muss erfindungsgemäß durch zusätzliche Maßnahmen eine Aerosolhauptströmung erzeugt werden. Im Folgenden wird ein Beispiel einer Verneblereinrichtung der zuletzt angesprochenen Art und geeignete Maßnahmen für die Erzeugung einer Aerosolhauptströmung geschildert.

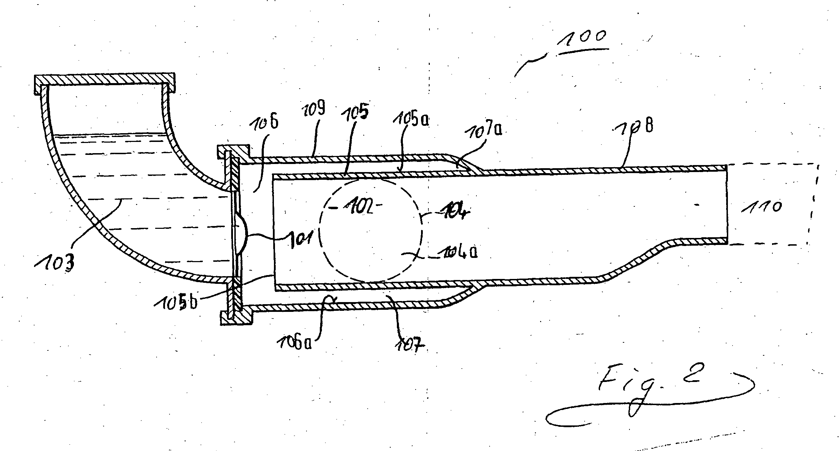

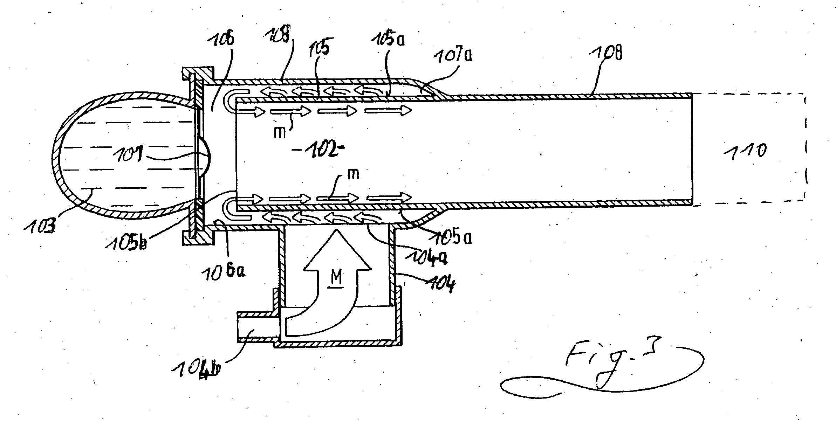

Dieses zweite Beispiel einer für die Anwendung der Erfindung geeigneten Verneblereinrichtung wird anhand der Figuren 2 und 3 erläutert, die beide die Verneblereinrichtung in einer geschnittenen Darstellung jedoch aus unterschiedlichen Blickrichtungen zeigen.

In den Figuren 2 und 3 ist eine Verneblereinrichtung 100 dargestellt, die einen Aerosolgenerator 101, beispielsweise einen Membran-Aerosolgenerator aufweist. Die zu vernebelnde Flüssigkeit wird in einem Flüssigkeitsreservoir 103 bevorratet und dem Aerosolgenerator 101 zugeführt. Der Aerosolgenerator 101 erzeugt aus der zugeführten Flüssigkeit ein Aerosol, das in einen Ausdehnungsraumbereich 102 hinein abgegeben wird. Insbesondere bei Membran-Aerosolgeneratoren erfolgt die Aerosolerzeugung und Abgabe in Form einer sich in gewissem Umfang gerichtet ausdehnenden Aerosolwolke, die sich vom Aerosolgenerator 101 aus in den Ausdehnungsbereich 102 hinein ausdehnt.

Die Verneblereinrichtung 100 umfasst ferner eine Zuführungseinrichtung 104 für die Zuführung eines unter Druck stehenden gasförmigen Mediums, bei dem es sich insbesondere um Luft, ein therapeutisch wirksames Gas oder ein für die Diagnostik geeignetes Gas handelt. Über die Zuführungseinrichtung 104 wird der Verneblereinrichtung 100 die Druckgasströmung so zugeführt, dass sich eine Aerosolhauptströmung einstellen kann, wie im Folgenden noch erläutert wird.

Das über die Zuführungseinrichtung 104 zugeführte Druckgas trifft bei der in den Figuren 2 und 3 gezeigten Verneblereinrichtung 100 auf eine Strömungsbeeinflussungseinrichtung 105. Der Strömungsverlauf des zugeführten gasförmigen Mediums M wird durch die Strömungsbeeinflussungseinrichtung 105 derart beeinflusst, dass das gasförmige Medium M einen Mantelstrom m um den Ausdehnungsbereich 102 für das Aerosol hervorruft und das Aerosol unter Ausbildung einer Aerosolhauptströmung zum Patienten transportiert. Der Strömungsverlauf des zugeführten gasförmigen Mediums M ist in Figur 3 in der Zeichnungsebene beispielhaft angedeutet. Dazu zeigt Figur 3 mehrere Pfeile, die das zugeführte Druckgas M repräsentieren, das sich um die Strömungsbeeinflussungseinrichtung 105 herum ausbreitet und diese so umströmt, dass es schließlich am Rand des Ausdehnungsraumbereichs 102 entlang strömt, in dem sich im Wesentlichen mittig die von dem Aerosolgenerator 101 abgegebene Aerosolwolke ausbreitet. Wie insbesondere aus Figur 3 unschwer zu entnehmen ist, stellt sich auf diese Weise die gewünschte in Richtung auf den Anschlussstutzen 108 für ein Nasenstück 110 gerichtete Aerosolhauptströmung ein, da das zugeführte Druckgas nur auf diesem Weg aus der Verneblereinrichtung 100 entweichen kann. Dabei nimmt es das erzeugte Aerosol mit und fördert es zum Anschlussstutzen 108 für das Nasenstück 110.

Bezüglich des Nasenstücks 110, das in den Figuren 2 und 3 nicht explizit dargestellt ist, wird auf die Beschreibung des Nasenstücks 10 aus Figur 1 weiter oben und auf die Darstellung in Figur 8 verwiesen.

Bei der in den Figuren 2 und 3 gezeigten Verneblereinrichtung ist die Strömungsbeeinflussungseinrichtung in Form eines zylindrischen Rohrstücks 105 realisiert, das um den Ausdehnungsraumbereich 102 angeordnet ist. Die vom Aerosolgenerator erzeugte Aerosolwolke dehnt sich in das zylindrische Rohrstück 105 hinein aus. Wie sich aus den Figuren 2 und 3 ferner ergibt, ist das zylindrische Rohrstück bei dem gezeigten Ausführungsbeispiel im Querschnitt kreisförmig, was zweckdienlich und vorteilhaft ist. Auf die äußere Manteloberfläche 105a des Rohstücks 105 trifft das zugeführte Druckgas M auf. Es strömt entlang der äußeren Manteloberfläche 105a um das zylindrische Rohrstück 105 herum und dehnt sich dabei in Richtung auf das stirnseitige Ende 105b des Rohrstücks 105 aus. Durch die Ab- und Umlenkung der Strömung des gasförmigen Mediums M wird das gasförmige Medium M im wesentlichen gleichmäßig um das zylindrische Rohrstück 105 herum verteilt und erreicht das stirnseitige Ende 105b, das dem Aerosolgenerator 101 gegenüber angeordnet ist, so dass das gasförmige Medium um das stirnseitige Ende 105b des Rohrstücks 105 herum strömt und im Randbereich der Ausdehnungszone 102 der Aerosolwolke entlang der inneren Oberfläche des Rohrstücks 5 weiterströmt. Dadurch bildet sich eine zylindrische Mantelströmung m um den Ausdehnungsraumbereich 102 für das Aerosol aus.

Wie in den Figuren 2 und 3 dargestellt, ist bei dem gezeigten Beispiel einer Verneblereinrichtung 100 das Rohrstück 105 einstückig mit dem Anschlussstutzen 108 für ein Nasenstück 110 ausgebildet, über das der Nase eines Patienten das in der Aerosolhauptströmung geförderte Aerosol zugeführt wird.

Bei dem in den Figuren 2 und 3 gezeigten Beispiel einer Verneblereinrichtung 100 ist das zylindrische Rohrstück 105 in einer Kammer 106 angeordnet, in die hinein der Aerosolgenerator 101 das Aerosol abgibt. Die Kammer 106 wird umschlossen von einem Abschnitt 109 des Gehäuses der Verneblereinrichtung 100, wobei der Gehäuseabschnitt 109, ähnlich wie das zylindrische Rohrstück 105, einen zylindrischen, beispielsweise einen kreiszylindrischen Querschnitt aufweist. Da der Spaltraum 107 in einem vom Aerosolerzeuger 101 entfernt angeordneten Bereich 107a abgeschlossen ist, findet die Ausbreitung des gasförmigen Mediums M in Richtung auf das stirnseitige Ende 105b des Rohrstücks 105 statt, das dem Aerosolgenerator 101 gegenüberliegt.

Aus den Figuren 2 und 3 ergibt sich für das erläuterte Beispiel einer Verneblereinrichtung 100 ferner, dass die Zuführeinrichtung für das gasförmige Medium M vorzugsweise ein zylindrischer Anschlussstutzen 104 ist, der an dem Gehäuse 109 der Verneblereinrichtung 100 vorgesehen ist und sich zur Kammer 106 hin öffnet. Die Austrittsöffnung 104a des zylindrischen Anschlussstutzens 104 ist dabei so angeordnet, dass sie auf das Rohrstück 105 ausgerichtet ist, so dass dieses ab- und umlenkend auf das gasförmige Medium M einwirkt.

In Figur 2 ist die Lage der Austrittsöffnung 104a bzw. des zylindrischen Anschlussstutzens 104 durch eine gestrichelte Linie angedeutet. Aus dieser Darstellung ergibt sich, dass der zylindrische Anschlussstutzen 104 vorzugsweise einen kreiszylindrischen Querschnitt aufweist und mittig zu dem zylindrischen Rohrstück 105 angeordnet ist. Jedoch kann der Anschlussstutzen 104 auch mit einem anderen Querschnitt, beispielsweise einem elliptischen Querschnitt ausgestattet und/oder außermittig zu dem Rohrstück 105 angeordnet sein.

Die Zuführeinrichtung 104 des gezeigten Beispiels einer Verneblereinrichtung 100 weist ferner ein Anschlusselement 104b für den Anschluss einer Zuleitung, beispielsweise eines Schlauchs auf. Über den Schlauch wird das Druckgas M zugeführt.

Die beiden zuvor geschilderten Beispiele zeigen, dass eine Verneblereinrichtung sich für den Einsatz im Rahmen einer eingangs geschilderten Aerosoltherapie eignet, wenn durch Zuführung eines Druckgases eine Aerosolhauptströmung hervorgerufen wird, die das von einem Aerosolgenerator der Verneblereinrichtung erzeugte Aerosol zu einem Nasenstück der Verneblereinrichtung transportiert, so dass es über das Nasenstück der Öffnung eines Nasenflügels eines Patienten zugeführt werden kann.

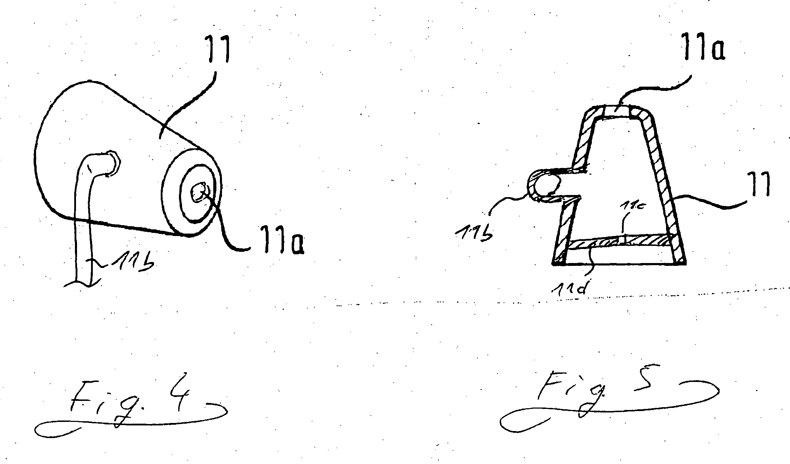

Gemäß einem Aspekt der hier geschilderten Erfindung ist in der Öffnung des anderen Nasenflügels des Patienten eine Strömungswiderstandseinrichtung 11 vorzusehen, die in den Figuren 4 und 5 gezeigt ist. Die Strömungswiderstandseinrichtung 11 umfasst eine erste Öffnung 11a, eine Anschlusseinrichtung 11b und einen Strömungswiderstand 11c, beispielsweise in Form einer zweiten Öffnung 11c, die kleiner ist als die erste Öffnung. Der so realisierte Strömungswiderstand ist größer als der Widerstand des natürlichen Strömungswegs durch die Nase des Patienten. Erst mit dem deutlich erhöhten Strömungswiderstand an der anderen Nasenöffnung und damit am Ende des Strömungsweges der Aerosolhauptströmung durch die Nase des Patienten wird erreicht, dass eine wirksame Menge des Aerosols in die Nasennebenhöhlen eindringt.

Die für das Eindringen des Aerosols in die Nasennebenhöhlen erforderlichen Druckschwankungen werden über die Anschlusseinrichtung 11b der Aerosolhauptströmung aufgeprägt. Dazu wird die Anschlusseinrichtung 11b an eine geeignete Quelle für Druckschwankungen angeschlossen.

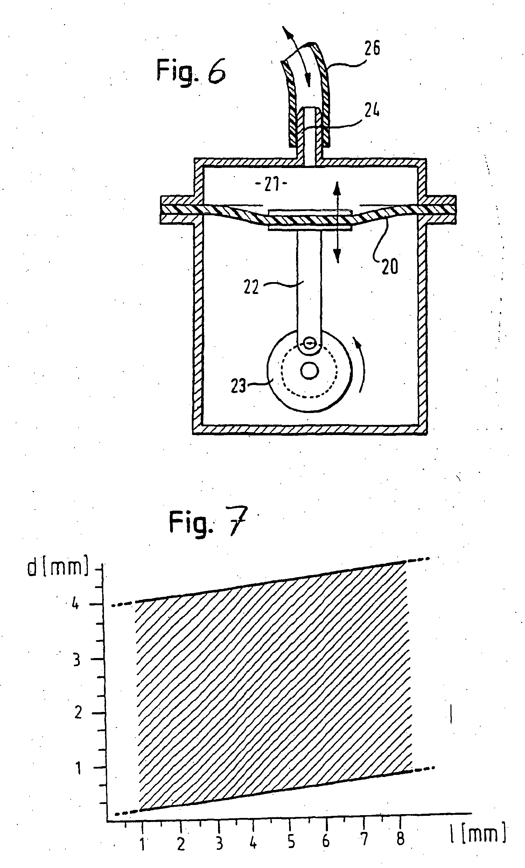

Die Druckschwankungen, die der Aerosolhauptströmung aufgeprägt-werden, können zum Beispiel auf verschiedene Art und Weise erzeugt werden. Wie in Fig. 6 gezeigt, können mit Hilfe einer Membran 20, die einen Hohlraum 21 (Druckkammer) druckdicht abschließt, die Druckschwankungen erzeugt werden, wenn die Membran 20 von einer Kolbenstange 22 hin und her bewegt wird. Die Kolbenstange 22 ist dazu an einer Antriebsscheibe 23 exzentrisch gelagert, so dass die Kolbenstange 22 eine die Druckschwankungen erzeugende Bewegung der Membran 20 bewirkt, wenn sich die Antriebsscheibe 23 dreht. Die Antriebsscheibe 23 wiederum ist dazu mit einem Elektromotor (nicht dargestellt) oder einem anderen geeigneten Antrieb verbunden.

Über eine an die Anschlusseinrichtung 11b angeschlossene Schlauchleitung gelangen die Druckschwankungen in das Innere der Strömungswiederstandseinrichtung 11 und durch die erste Öffnung 11a in die Nasenhöhlen des Patienten.

Die Strömungswiderstandseinrichtung 11 für eine Nasenöffnung des Patienten kann zum Beispiel in Form des in den Figuren 4 und 5 gezeigten Stopfens 11 mit einer großen ersten Öffnung 11a und einer kleinen zweiten Öffnung 11c, dem eigentlichen Strömungswiderstand, realisiert werden. Der Stopfen besitzt eine konische Grundform mit einem Öffnungswinkel a im Bereich von 10° bis 40°, die an die Nasenöffnungen der menschlichen Nase angepasst ist und dadurch einen sicheren Sitz gewährleistet. Der Stopfen ist vorzugsweise hohl, wie in Fig. 5 erkennbar, und besitzt am verjüngten Ende die erste Bohrung 11a, die die Nasenhöhlen des Patienten mit dem Inneren des Stopfens verbindet. In den Innenraum mündet auch die Anschlusseinrichtung 11b für die Druckschwankungen. Nach Außen ist der Innenraum durch eine Wand 11d abgeschlossen, die lediglich die kleinere zweite Bohrung 11c aufweist. Der Durchmesser d der zweiten Bohrung und ihre Länge 1 bestimmen den Strömungswiderstand, der der Aerosolhauptströmung und der Druckschwankungsströmung entgegen gesetzt wird. Fig. 7 zeigt beispielhaft einen Bereich möglicher Wertepaarungen von Durchmesser d und Länge 1 der zweiten Öffnung 11c einer erfindungsgemäßen Strömungswiderstandseinrichtung. Aus diesem Bereich der in Figur 7 grau hinterlegt ist, können geeignete Werte für d und 1 entnommen werden.

Um die Handhabung des Nasenstücks 10 zu vereinfachen und um einem Verlust des Stopfens 11 entgegen zu wirken, ist der Stopfen 11 vorzugsweise mit dem Nasenstück 10 verbunden, wie Figur 8 zeigt. Dies kann durch ein flexibles Verbindungselement 13 erreicht werden, was die einstückige Ausgestaltung des Nasenstücks 10 und der Strömungswiderstandseinrichtung 11 aus ein und demselben Material nahe legt.

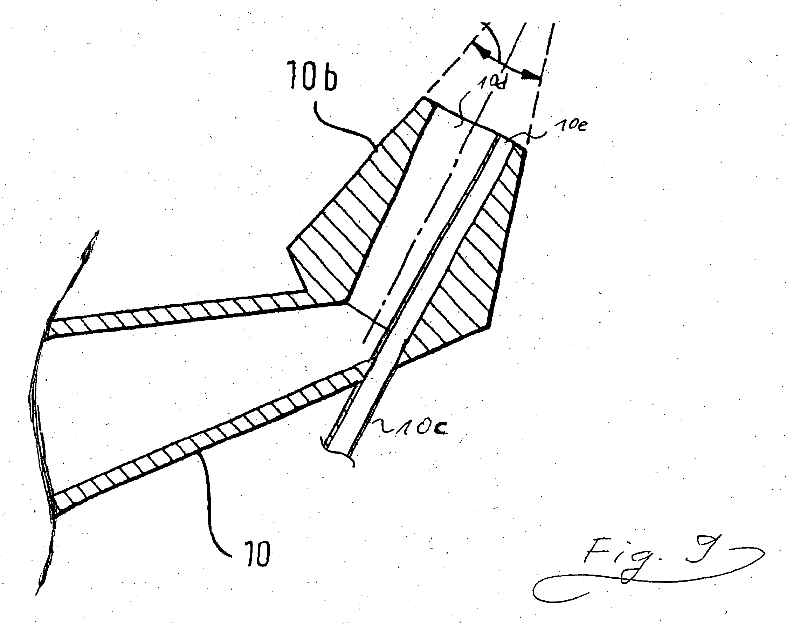

Gemäß einem anderen Aspekt der hier geschilderten Erfindung ist, wie Figur 9 zeigt, das Nasenstück 10 mit einer Anschlusseinrichtung 10c für die Zuführung von Druckschwankungen ausgestattet. Mit anderen Worten, die für das Eindringen des Aerosols in die Nasennebenhöhlen erforderlichen Druckschwankungen werden an der Nasenöffnung der Aerosolhauptströmung aufgeprägt, an der auch die Aerosolhauptströmung appliziert wird. Die Anschlusseinrichtung 10c erstreckt sich dazu bis zu einer Hauptaustrittsöffnung 10d des Nasenstücks 10, aus der die Aerosolhauptströmung austritt. Dort ist in unmittelbar Nähe zur Hauptaustrittsöffnung 10d des Nasenstücks 10 die Austrittsöffnung 10e der Anschlusseinrichtung 10c angeordnet. Die über die Anschlusseinrichtung 10c zugeführten Druckschwankungen werden so der Aerosolhauptströmung überlagert. Dazu wird die Anschlusseinrichtung 10c an eine geeignete Quelle für Druckschwankungen angeschlossen. Hinsichtlich der Quelle für Druckschwankungen wird auf die Beschreibung der Figur 6 verwiesen.

Bei dem zuletzt geschilderten Aspekt der Erfindung wird zweckdienlicherweise den Druckschwankungen, die über die Anschlusseinrichtung 10c der Aerosolhauptströmung überlagert werden, eine Grundströmung hinzugefügt, indem eine zweite Druckgasströmung über die Anschlusseinrichtung 10c zugeführt wird. Die zusätzliche Druckgasströmung ist deutlich geringer als die Druckgasströmung, die für die Erzeugung des Aerosolhauptstroms durch die Nasenhöhlen des Patienten erforderlich ist. Das Verhältnis der Flüsse zwischen der Aerosolhauptströmung und dem überlagerten Zusatzfluss beträgt vorzugsweise mindestens 2:1.

Beiden Aspekten der Erfindung ist gemein, dass die Zuführung der Druckschwankungen unmittelbar an der einen oder der anderen Nasenöffnung der Nase des Patienten erfolgt. Gemäß dem einen Aspekt der Erfindung erfolgt dies durch Aufprägung der Druckschwankungen über den Stopfen, der den Strömungswiderstand an der Nasenöffnung darstellt, aus der die Aerosolhauptströmung austritt. Gemäß dem anderen Aspekt der Erfindung werden die Druckschwankungen über das Ende des Nasenstücks in die Nasenhöhlen des Patienten eingebracht und der Aerosolhauptströmung überlagert; dabei ist das Nasenstück ähnlich wie die Strömungswiderstandseinrichtung in Form eines Stopfens für die Einbringung in die Nasenöffnung ausgebildet.

Bei beiden Aspekten der Erfindung kann die Deposition des Aerosols in den Nasennebenhöhlen dadurch verbessert werden, dass die Druckschwankungen getaktet werden. Mit anderen Worten, die Druckschwankungen werden der Aerosolhauptströmung nicht permanent überlagert, sondern nur in ausgewählten Zeiträumen. Die Triggerung bzw. zeitliche Taktung der aufgeprägten Druckschwankungen kann so erfolgen, dass erst nach einer vorbestimmten Zeitspanne nach Einsetzen der Aerosolerzeugung und der Aerosolhauptströmung die Überlagerung der Druckschwankungen erfolgt. Zu diesem Zeitpunkt sind die Nasenhöhlen des Patienten mit Aerosol gefüllt.

Aus der vorangegangenen Beschreibung der Erfindung anhand von Ausführungsbeispielen ist unschwer erkennbar, dass ein wesentlicher Vorteil der erfindungsgemäßen Gerätekonfiguration darin besteht, dass man damit gezielt Krankheiten des oberen und unteren Respirationstrakts behandeln kann, wenn diese eine nasale oder paranasale Ursache haben. Außerdem ergibt sich für den fachkundigen Leser, dass sich eine Gerätekonfiguration gemäß der Erfindung dadurch auszeichnet, dass flüssige Arzneiformulierungen damit besonders vorteilhaft in Form eines Aerosolnebels in die paranasalen Kavitäten deponiert werden können, um Krankheiten zu behandeln, die dort ihren Ursprung haben.

Diese Charakteristika der erfindungsgemäßen Gerätekonfiguration führen dazu, dass durch gezielte Deposition von Wirkstoffen in die Nase und paranasalen Kavitäten, Krankheiten wie chronische oder allergische Sinusitis, Entzündungen oder Infektionen oder andere Krankheits- oder Befindlichkeitszustände ("Stinkenase") topisch behandelt werden können, so dass auf diesem Weg unerwünschte Nebenwirkungen einer systemischen Arzneitherapie vermieden werden können.

Sinn und Zweck der erfindungsgemäßen Aerosolvorrichtung ist es, Wirkstoffe gezielt in die höhlenförmigen Kavitäten im Bereich der Nase und Stirnhöhle zu bringen. Diese sind aufgrund anatomischer Gegebenheiten schlecht durchblutet und sehr oft schlecht durchlüftet weshalb Wirkstoffe, die oral oder parenteral appliziert werden, nicht in therapeutisch effektiven Konzentrationen an den Wirkort gelangen. Da die Zugänge sehr klein und häufig verlegt sind, können bevorzugt nur solche Medikamentenformulierungen an den Zielort gelangen, die mit Aerosoltröpfchen transportiert werden, die einen Durchmesser kleiner 10 µm und bevorzugt etwa 2 bis 5 µm aufweisen. Der therapeutische Effekt kann durch die Verwendung von oberflächenaktiven und adhäsiven Hilfsstoffen in den Wirkstoffformulierungen verbessert werden, weil durch diese Hilfsstoffe deren Spreizbarkeit und Benetzungsfähigkeit verbessert wird. Zur Abschwellung der Nasenschleimhaut bietet es sich an vasokonstriktive Substanzen vorher oder in Kombination mit entzündungshemmenden und antiallergischen Wirkstoffen, wie z.B. Kortikoiden und/oder Antibiotika zu applizieren.

Unter den Wirkstoffen, die zum Erreichen einer dieser Zwecke nützlich sein können, sind zum Beispiel Substanzen, die aus den Gruppen der Entzündungshemmer, Glucokortikoide, Antiinfektiva, Antibiotika, Fungizide, Viruzide allein oder in Kombination mit Biofilm-reduzierenden Verbindungen oder Inhibitoren von Efflux-Pumpen, Antiseptika, Immunmodulatoren, Antioxidanzien, Mykolytika, Dekongestiva, Vasokonstriktoren, Wundheilmittel, Lokalanästhetika, Peptide, Proteine und natürliche oder künstliche Pflanzenextrakte ausgewählt werden.

Beispiele von potentiell nützlichen Entzündungshemmern sind steroidale Wirkstoffe wie Glucokortikoide wie Betamethason, Beclomethason, Budesonid, Ciclesonid, Dexamethason, Desoxymethason, Fluoconolonacetonid, Flucinonid, Flunisolid, Fluticason, Icomethason, Rofleponide, Triamcinolonacetonid, Fluocortinbutyl, Hydrocortison, Hydroxycortison-17-butyrat, Prednicarbat, 6-Methylprednisolonaceponat, Mometasonfuroat und nicht-steroidale Entzündungshemmer (NSAIDs) wie Prostaglandin-, Leukotrien-, Elastase-, Bradykinin-Antagonisten, Heparin und Heparinoide, nicht-glucokortikoale Steroide wie Dehydroepiundrostendieone und Dehdropianthrosterone (DHEA); irgendwelche pharmazeutisch akzeptablen Salze, Ester, Isomere, Stereoisomere, Diasteriomere, Epimere, Solvate oder andere Hydrate, Pro-Pharmaka, Derivate oder irgendwelche anderen chemischen oder physikalischen Formen der Wirkstoffe, die die entsprechenden aktiven Einheiten umfassen.

Beispiele von Antiinfektiva, deren Klasse oder therapeutische Kategorie hierin so verstanden wird, dass sie Verbindungen umfasst, die gegen bakterielle, pilzartige und virale Infektionen wirksam sind, d.h., dass sie die Klassen der antimikrobiellen Substanzen, der Antibiotika, der Fungizide, der Antiseptika und der Viruzide beinhaltet, allein oder in Kombination mit Biofilm-reduzierenden oder - hemmenden Mitteln und Inhibitoren der Efflux-Pumpe, sind
- Penicilline, inklusive Benzylpenicilline (Penicillin-G-sodium, Clemizonpenicillin, Benzathinpenicillin G), Phenoxypenicilline (Penicillin V, Propicillin), Aminobenzylpenicilline (Ampicillin, Amoxycillin, Bacampicillin), Acylaminopenicilline (Azlocillin, Mezlocillin, Piperacillin, Apalcillin), Carboxypenicilline (Carbenicillin, Ticarcillin, Temocillin), Isoxazolylpenicilline (Oxacillin, Cloxacillin, Dicloxacillin, Flucloxacillin), und Amiidinpenicilline (Mecillinam);
- Cephalosporine, inklusive Cefazoline (Cefazolin, Cefazedone); Cefuroxime (Cerufoxim, Cefamdole, Cefotiam), Cefoxitine (Cefoxitin, Cefotetan, Latamoxef, Flomoxef), Cefotaxime (Cefotaxim, Ceftriaxon, Ceftizoxim, Cefmenoxim), Ceftazidime (ceftazidim, Cefpirom, Cefepim), Cefalexin (Cefalexin, Cefaclor, Cefadroxil, Cefradin, Loracarbef, Cefprozil), und Cefixime (Cefixim, Cefpodoximproxetil, Cefuroximaxetil, Cefetametpivoxil, Cefotiamhexetil), Loracarbef, Cefepim, Clavulansäure / Amoxicillin, Ceftobiprole;
- Synergisten, inklusive Beta-Lactamaseinhibitoren, wie Clavulansäure, Sulbactam, und Tazobactam;
- Carbapeneme, inklusive Imipenem, Cilastin, Meropenem, Doripenem, Tebipenem, Ertapenem, Ritipenam, und Biapenem;
- Monobactame, inklusive Aztreonam;
- Aminoglycoside, wie Apramycin, Gentamicin, Amikacin, Isepamicin, Arbekacin, Tobramycin, Netilmicin, Spectinomycin, Streptomycin, Capreomycin, Neomycin, Paromoycin, und Kanamycin;
- Macrolide, inklusive Erythromycin, Clarythromycin, Roxithromycin, Azithromycin, Dithromycin, Josamycin, Spiramycin und Telithromycin;
- Gyraseinhibitoren oder Fluroquinolone, inklusive Ciprofloxacin, Gatifloxacin, Norfloxacin, Ofloxacin, Levofloxacin, Perfloxacin, Lomefloxacin, Fleroxacin, Garenoxacin, Clinafloxacin, Sitafloxacin, Prulifloxacin, Olamufloxacin, Caderofloxacin, Gemifloxacin, Balofloxacin, Trovafloxacin, und Moxifloxacin;
- Tetracycline, inklusive Tetracyclin, Oxytetracyclin, Rolitetracyclin, Minocyclin, Doxycycline, Tigecycline und Aminocycline;
- Glycopeptide, inklusive Vancomycin, Teicoplanin, Ristocetin, Avoparcin, Oritavancin, Ramoplanin, und Peptid 4;
- Polypeptide, inklusive Plectasin, Dalbavancin, Daptomycin, Oritavancin, Ramoplanin, Dalbavancin, Telavancin, Bacitracin, Tyrothricin, Neomycin, Kanamycin, Mupirocin, Paromomycin, Polymyxin B und Colistin;
- Sulfonamide, inklusive Sulfadiazin, Sulfamethoxazol, Sulfalen, Co-Trimoxazole, Co-Trimetrol, Co-Trimoxazin, und Co-Tetraxazin;
- Azole, inklusive Clotrimazol, Oxiconazol, Miconazol, Ketoconazol, Itraconazol, Fluconazol, Metronidazol, Tinidazol, Bifonazol, Ravuconazol, Posaconazol, Voriconazol, und Ornidazol und andere Fungizide inklusive Flucytosin, Griseofluvin, Tonoftal, Naftifin, Terbinafin, Amorolfin, Ciclopiroxolamin, Echinocundins, wie Micafungin, Caspofungin, Anidulafungin;
- Nitrofurane, inklusive Nitrofurantoin und Nitrofuranzon;
- Polyene, inklusive Amphotericin B, Natamycin, Nystatin, Flucocytosin;
- andere Antibiotika, inklusive Tithromycin, Lincomycin, Clindamycin, Oxazolindione (Linzezolide), Ranbezolid, Streptogramin A+B, Pristinamycin aA+B, Virginiamycin A+B, Dalfopristin/Giunupristin (Synercid), Chloramphenicol, Ethambutol, Pyrazinamid, Terizidon, Dapson, Prothionamid, Fosfomycin, Fucidinicsäure, Rifampicin, Isoniazid, Cycloserine, Terizidone, Ansamycin, Lysostaphin, Iclaprim, Mirocin B17, Clerocidin, Filgrastim, und Pentamidin;
- Viruzide, inklusive Aciclovir, Ganciclovir, Birivudin, Valaciclovir, Zidovudine, Didanosin, Thiacytidin, Stavudin, Lamivudin, Zalcitabin, Ribavirin, Nevirapirin, Delaviridin, Trifluridin, Ritonavir, Saquinavir, Indinavir, Foscarnet, Amantadin, Podophyllotoxin, Vidarabin, Tromantadin, und Proteinaseinhibitoren;
- Antiseptica, inklusive Acridinderivative, Jod-Povidon, Benzoate, Rivanol, Chlorhexidin, quarternäre Ammoniumverbindungen, Cetrimide, Biphenylol, Clorofen, und Octenidine;
- Pflanzenextrakte oder -bestandteile, wie Pflanzenextrakte der Kamille, Hamamelis, Echinacea, Calendula, Papain, Pelargonium, essentielle Öle, Myrtol, Pinen, Limonen, Cineol, Thymol, Mentol, Kampfer, Tannin, Alpha-Hederin, Bisabolol, Lycopodin, Vitapherol,
- wundheilende Verbindungen inklusive Dexpantenol, Allantoin, Vitamine, Hyaluronsäure, Alpha-Antitrypsin, anorganische und organische Zinksalze/verbindungen, Wismuthsalze, Interferone (alpha, beta, gamma), Tumornecrosefaktoren, Cytokine, Interleukine, Immunmodulatoren inclusive Methotrexat, Azathioprin, Cyclosporin, Tacrolimus, Sirolimus, Rapamycin, Mofetil, Cytostatika und Metastaseinhibitoren, Alkylanten, wie Nimustin, Melphanlan, Carmustin, Lomustin, Cyclophosphosphamid, Ifosfamid, Trofosfamid, Chlorambucil, Busulfan, Treosulfan, Prednimustin, Thiotepa;
- Antimetaboliten, z. B. Cytarabin, Fluorouracil, Methotrexat, Mercaptopurin, Tioguanin; Alkaloide, wie Vinblastin, Vincristin, Vindesin; Antibiotika, wie Alcarubicin, Bleomycin, Dactinomycin, Daunorubicin, Doxorubicin, Epirubicin, Idarubicin, Mitomycin, Plicamycin; Komplexe von Zweitgruppenelementen (z.B. Ti, Zr, V, Nb, Ta, Mo, W, Pt) wie Carboplatin, Cis-Platin und Metallocenverbindungen wie Titanocendichlorid; Amsacrin, Dacarbazin, Estramustin, Etoposid, Beraprost, Hydroxycarbamid, Mitoxanthron, Procarbazin, Temiposid; Paclitaxel, Iressa, Zactima, Poly-ADP-Ribose-Polymerase (PRAP) Enzyminhibitoren, Banoxantron, Gemcitabin, Pemetrexed, Bevacizumab, Ranibizumab.

Beispiele von potentiell nützlichen Mykolytika sind DNase, P2Y2-Agonisten (Denufosol), Heparinoide, Guaifenesin, Acetylcystein, Carbocystein, Aambroxol, Bromhexin, Tyloxapol, Lecithine, Myrtol, und rekombinante oberflächenaktive Proteine.

Beispiele von potentiell nützlichen Vasokonstriktoren, die nützlich sein können, um das Anschwellen der Mukosa zu verringern, sind Phenylephrin, Naphazolin, Tramazolin, Tetryzolin, Oxymetazolin, Fenoxazolin, Xylometazolin, Epinephrin, Isoprenalin, Hexoprenalin, und Ephedrin.

Beispiele von potentiell nützlichen Lokalanästhetika beinhalten Benzocain, Tetracain, Procain, Lidocain und Bupivacaine.

Beispiele von potentiell nützlichen Antiallergika beinhalten die oben erwähnten Glucokortikoide, Cromolynnatrium, Nedocromil, Cetrizin, Loratidin, Montelukast, Roflumilast, Ziluton, Omalizumab, und Heparinoide.

Beispiele von potentiell nützlichen Peptiden und Proteinen beinhalten von Mikroorganismen hergestellte Antikörper gegen Toxine, antimikrobielle Peptide wie Cecropine, Defensine, Thionine, und Cathelicidine.

Kombinationen von irgendeinem der oben erwähnten Wirkstoffe, die aus irgendeinem pharmazeutische akzeptablen Salz, Ester, Isomer, Stereoisomer, Diastereomer, Epimer, Solvat oder anderen Hydrat, Pro-Pharmakon, Derivat oder irgendeiner anderen chemischen oder physikalischen Form von Wirkstoffen bestehen, die die entsprechenden aktiven Einheiten umfassen.

Oben genannte Wirkstoffe werden vorzugsweise in Form ihrer pharmazeutisch gebräuchlichen Konfigurationen oder als Salze, Ester, Isomere, Stereoisomere, Diastereomere, Epimere etc. verwendet mit der Zielsetzung, jeweils eine lagerungsstabile Darreichungsform zu erhalten. Hierzu können Formulierungen in den verschiedensten Darreichungsformen, wie z.B. als Lösungen Suspensionen, Emulsionen, Pulver, oder Lyophilisate u.a. in 2 Kammersystemen mit wässrigen und nicht wässrigen Lösungsmittel oder Gemischen etc. verwendet werden. Von Vorteil ist der Zusatz von Hilfsstoffen, die die Löslichkeit verbessern, wie z.B. Glycerol, Propylenglycol, Ethanol, die Penetration in Nasenneben- und Stirnhöhlen fördern, die Oberflächenspannung vermindern und/oder die Anlagerungszeit und Verweilzeit (control release) am Bestimmungsort verlängern, was z.B. durch den Zusatz von nichtionischen Tensiden, wie. z.B. Tyloxapol, Vitamin E-TPGS, Polysorbaten, Pluronics, etc. und/oder anderer Additive wie z.B. Phospholipide, Celluoseether, Dextrane, Chitosane, Cyclodextrine, Polyvinylpyrrolidon, Polyvinylalkohol, etc. erreicht werden kann.

Die Formulierung und Applikation der oben aufgeführten Wirkstoffklassen und Substanzen als Liposomen, Suspensionen und Emulsionen erfolgt im Mikrometerbereich und bevorzugt im Nanometerbereich, mit einem geometrischen Durchmesser kleiner ca. 1 µm, die besonders geeignet sind von kleinen Tröpfchen transportiert zu werden. Damit wird sichergestellt, dass diese Zubereitungen mittels der erfindungsgemäßen Vorrichtung besser in die Nasenneben- und Stirnhöhlen penetrieren und deponieren und dadurch ihre Wirkung entfalten können. Wirkstoffe, die aufgrund unzureichender Lagerungsstabilität in Lösung als feste Formulierungen eingesetzt werden müssen, können kurz vor der Anwendung mit einem geeigneten wässrigen oder nicht wässrigen Lösungsmitteln (wie z.B. Glycerol, Propylenglycol, Polyglykolen, Pluronics, Ethanol) oder Gemischen hieraus entweder gelöst oder suspendiert werden. Beansprucht wird des weiteren ein Umhüllungs- und Einschlussverfahren, um schlecht riechende oder lokal irritierende Substanzen durch Komplexierung z.B. mit Cyclodextrinen für die Applikation verträglicher zu machen. Alternativ können diese Wirkstoffe auch an polymere Hilfsstoffe, wie z.B. Chitosan- und Celluloseetherderivate oder Gelatine gebunden werden, um die Ab- und Absorptionseigenschaften so zu modifizieren, damit der therapeutische Effekt verstärkt und die Applikationshäufigkeit reduziert werden kann. Vorteilhaft ist die Verwendung von iso- oder hypertonen Lösungen, die lösliche Alkali- und Erdalkalisalze enthalten (wie z.B. Emser Sole, Magnesiumchlorid, Natriumhydrogencarbonat, etc.) und einen physiologischen pH-Bereich (4 - 9) aufweisen. Dies kann durch den Zusatz von pharmazeutisch gebräuchlichen Puffersubstanzen zu den Wirkstoffformulierungen erreicht werden. Die Formulierungen können auch mit entsprechenden pharmazeutisch geeigneten Aroma- und Geschmackskorrigentien versehen werden, um deren Akzeptanz insbesondere bei Kindern zu verbessern.

Die erfindungsgemäße Aerosoltherapievorrichtung ist ferner dadurch gekennzeichnet, dass man damit gezielt Krankheiten des oberen und unteren Respirationstrakts behandeln kann, wenn diese eine nasale oder paranasale Ursache haben.

Die erfindungsgemäße Aerosoltherapievorrichtung ist ferner dadurch gekennzeichnet, dass flüssige Arzneiformulierungen damit besonders vorteilhaft in Form eines Aerosolnebels in die paranasalen Kavitäten deponiert werden können, um Krankheiten zu behandeln, die dort ihren Ursprung haben.

Die erfindungsgemäße Aerosoltherapievorrichtung ist ferner dadurch gekennzeichnet, dass durch gezielte Deposition von Wirkstoffen in die Nase und paranasalen Kavitäten, Krankheiten wie chronische oder allergische Sinusitis, Entzündungen oder Infektionen oder andere Krankheits- oder Befindlichkeitszustände ("Stinkenase") topisch behandelt werden, um dadurch unerwünschte Nebenwirkungen einer systemischen Arzneitherapie zu vermeiden.

## Patentansprüche

1. Aerosoltherapievorrichtung mit
a) einer Verneblereinrichtung (1, 100), der ein Druckgas, vorzugsweise Druckluft zuführbar ist, mit einem Aerosolgenerator (2, 102) für die Erzeugung eines Aerosols, das zusammen mit dem zugeführten Druckgas eine Aerosolhauptströmung ausbildet,
b) einem Nasenstück (10), das mit der Verneblereinrichtung (1, 100) verbunden ist, für die Zuführung des Aerosols zu einer der beiden Nasenöffnungen eines Benutzers,
c) einer Strömungswiderstandseinrichtung (11) für die Bereitstellung eines Strömungswiderstandes in der anderen der beiden Nasenöffnungen des Benutzers, und
d) einer Anschlusseinrichtung (10c, 11b) für die Zuführung von Druckschwankungen, die der Aerosolhauptströmung überlagert werden, wobei die Anschlusseinrichtung (10c, 11b) derart an dem Nasenstück (10) oder der Strömungswiderstandseinrichtung (11) ausgebildet ist, dass die Druckschwankungen unmittelbar in die jeweilige der beiden Nasenöffnungen des Benutzers eingebracht werden.

2. Aerosoltherapievorrichtung nach Anspruch 1, **dadurch gekennzeichnet, dass** die Strömungswiderstandseinrichtung (11) die Anschlusseinrichtung (11b) für die Zuführung von Druckschwankungen, die sich in einen Innenraum der Strömungswiderstandseinrichtung erstreckt, umfasst und ferner eine erste Öffnung (11a) für die Verbindung des Innenraums mit der Nasenhöhle des Benutzers aufweist.

3. Aerosoltherapievorrichtung nach Anspruch 1 oder 2, **dadurch gekennzeichnet, dass** die Strömungswiderstandseinrichtung (11) eine zweite Öffnung (11c) aufweist, die derart gestaltet ist, dass der Strömungswiderstand größer ist als der Strömungswiderstand des natürlichen Strömungswegs durch die Nasenhöhlen des Benutzers.

4. Aerosoltherapievorrichtung nach Anspruch 2, **dadurch gekennzeichnet, dass** die zweite Öffnung (11c) in einer Wand der Strömungswiderstandseinrichtung (11) ausgebildet ist, die den Innenraum der Strömungswiderstandseinrichtung (11) begrenzt.

5. Aerosoltherapievorrichtung nach einem der vorangegangenen Ansprüche, **dadurch gekennzeichnet, dass** die Strömungswiderstandseinrichtung (11) ein Stopfen zum Einführen in die Nasenöffnung ist.

6. Aerosoltherapievorrichtung nach Anspruch 5, **dadurch gekennzeichnet, dass** der Stopfen (11) in Form eines Kegelstumpfes, vorzugsweise mit einem Öffnungswinkel a im Bereich von 10 bis 40° ausgebildet ist.

7. Aerosoltherapievorrichtung nach Anspruch 1, **dadurch gekennzeichnet, dass** das Nasenstück (10) eine Anschlusseinrichtung (10c) für die Zuführung von Druckschwankungen aufweist, wobei die Anschlusseinrichtung eine Austrittsöffnung (10e) aufweist, die im Bereich der Hauptaustrittsöffnung (10d) des Nasenstücks (10) für die Aerosolhauptströmung angeordnet ist.

8. Aerosoltherapievorrichtung nach Anspruch 7, **dadurch gekennzeichnet, dass** das Nasenstück (10) an einem Ende (10a) für die Befestigung an einem Anschlussstutzen (8, 108) der Verneblereinrichtung (1, 100) und am anderen Ende (10b) für das Einführen in eine Nasenöffnung und das dichte Verschließen der einen Nasenöffnung eines Benutzers ausgebildet ist.

9. Aerosoltherapievorrichtung nach Anspruch 8, **dadurch gekennzeichnet, dass** das für das Einführen in eine Nasenöffnung ausgebildete Ende (10b) des Nasenstücks (10) in Form eines Kegelstumpfes, vorzugsweise mit einem Öffnungswinkel a im Bereich von 10° bis 40° ausgebildet ist.

10. Aerosoltherapievorrichtung nach einem der vorangegangenen Ansprüche, **dadurch gekennzeichnet, dass** die Strömungswiderstandseinrichtung (11) durch ein Verbindungselement (13) mit dem Nasenstück (10) verbunden ist.

11. Aerosoltherapievorrichtung nach einem der vorangegangenen Ansprüche, **dadurch gekennzeichnet, dass** die Frequenz der Druckschwankungen im Bereich von 10 bis 100 Hz, vorzugsweise im Bereich von 15 bis 55 Hz liegen.

12. Aerosoltherapievorrichtung nach einem der vorangegangenen Ansprüche, **dadurch gekennzeichnet, dass** die Druckschwankungen erzeugt werden mittels eines Membran-Kompressors, der eine Membran (21) aufweist, die eine Druckkammer (20) druckdicht verschließt und die von einer Kolbenstange hin und her bewegt wird.

13. Aerosoltherapievorrichtung nach einem der vorangegangenen Ansprüche, **dadurch gekennzeichnet, dass** der Aerosolgenerator eine Verneblerdüse (2) mit einem Druckluftkanal (5), der in eine Düsenöffnung (6) mündet, und mit zumindest einem Ansaugkanal (7) ist, durch den eine zu vernebelnde Flüssigkeit angesaugt wird, wobei das der Verneblereinrichtung zugeführte Druckgas auch die Erzeugung des Aerosols bewirkt.

14. Aerosoltherapievorrichtung nach einem der vorangegangenen Ansprüche 1 bis 12, **dadurch gekennzeichnet, dass** der Aerosolgenerator ein Membran-Aerosol-Generator (101) ist und die Verneblereinrichtung (100) ferner eine Zuführeinrichtung (104, 104a, 104b) für die Zuführung des Druckgases (M) zur Erzeugung einer Aerosolhauptströmung aufweist.

15. Aerosoltherapievorrichtung nach Anspruch 14, **dadurch gekennzeichnet, dass** die Verneblereinrichtung (100) ein zylindrisches Rohrstück (105) umfasst, das um den Ausdehnungsraumbereich (102), in dem sich das von dem Aerosolgenerator erzeugte Aerosol ausbreitet, derart angeordnet ist, dass das zugeführte Druckgas (M) auf
die äußere Manteloberfläche (105a) des Rohrstücks (105) auftrifft und an einem stirnseitigen Ende (105b) in das Innere des Rohrstücks (105) strömt.

16. Aerosoltherapievorrichtung nach Anspruch 15, **dadurch gekennzeichnet, dass** das zylindrische Rohrstück (105) in einer von der Verneblereinrichtung umschlossenen Kammer (106) derart angeordnet ist, dass zwischen einer äußeren Manteloberfläche (105a) des zylindrischen Rohrstücks (105) und einer inneren Wandoberfläche (106a) der Kammer (106) ein Spaltraum (107) für die Ausbreitung des zugeführten Druckgases (M) ausgebildet ist.

17. Aerosoltherapievorrichtung nach Anspruch 15 oder 16, **dadurch gekennzeichnet, dass** das stirnseitige Ende (105b) des zylindrischen Rohrstücks (105) vor dem Aerosolgenerator (101) angeordnet ist.

18. Aerosoltherapievorrichtung nach einem der Ansprüche 15 bis 17, **dadurch gekennzeichnet, dass** das zylindrische Rohrstück (105) kreiszylinderförmig ist.

19. Aerosoltherapievorrichtung nach einem der Ansprüche 14 bis 18, **dadurch gekennzeichnet, dass** die Zuführeinrichtung für das Druckgas (M) ein zylindrischer Anschlussstutzen (104) ist.

20. Aerosoltherapievorrichtung nach Anspruch 19, **dadurch gekennzeichnet, dass** der zylindrische Anschlussstutzen (104) eine zu dem zylindrischen Rohrstück (105) gerichtete Austrittsöffnung (104a) aufweist.

21. Aerosoltherapievorrichtung nach Anspruch 20, **dadurch gekennzeichnet, dass** die Zuführeinrichtung (104) für das Druckgas (M) eine Anschlusseinrichtung (104b) für den Anschluss einer Zuführleitung für das Druckgas (M) aufweist.

22. Aerosoltherapievorrichtung nach einem der vorangegangenen Ansprüche 1 bis 21, **dadurch gekennzeichnet, dass** die Zuführung des Druckgases nur in vorbestimmten Zeiträumen erfolgt.

23. Aerosoltherapievorrichtung nach einem der vorangegangenen Ansprüche 1 bis 22, **dadurch gekennzeichnet, dass** den zugeführten Druckschwankungen eine zusätzliche Druckgasströmung hinzugefügt wird, die kleiner ist als die Druckgasströmung für die Erzeugung der Aerosolhauptströmung.

## Claims

1. Aerosol treatment device having
a) a nebuliser device (1, 100), to which a compressed gas, preferably compressed air, can be supplied, having an aerosol generator (2, 102) for the generation of an aerosol, which together with the supplied compressed gas, forms a main aerosol flow,
b) a nosepiece (10), which is connected to the nebuliser device (1, 100), for supplying the aerosol to one of the two nares of a user,
c) a flow resistance device (11) for providing a flow resistance in the other of the two nares of the user, and
d) a connection device (10c, 11b) for supplying pressure variations which are superimposed on the main aerosol flow, wherein the connection device (10c, 11b) is formed on the nosepiece (10) or the flow resistance device (11) such that the pressure variations are introduced directly into the particular one of the two nares of the user.

2. Aerosol treatment device according to claim 1, **characterised in that** the flow resistance device (11) comprises the connection device (11b) for supplying pressure variations which extends into an inner chamber of the flow resistance device and furthermore has a first opening (11a) for connection of the inner chamber with the nasal cavity of the user.

3. Aerosol treatment device according to claim 1 or 2, **characterised in that** the flow resistance device (11) has a second opening (11c) which is designed such that the flow resistance is greater than the flow resistance of the natural flow path through the nasal cavities of the user.

4. Aerosol treatment device according to claim 2, **characterised in that** the second opening (11c) is formed in one wall of the flow resistance device (11) which defines the inner chamber of the flow resistance device (11).

5. Aerosol treatment device according to one of the preceding claims, **characterised in that** the flow resistance device (11) is a stopper for introduction into the naris.

6. Aerosol treatment device according to claim 5, **characterised in that** the stopper (11) is designed in the form of a frustum, preferably with an aperture angle a in the range from 10 to 40°.

7. Aerosol treatment device according to claim 1, **characterised in that** the nosepiece (10) has a connection device (10c) for the supply of pressure variations, wherein the connection device has an outlet opening (10e) which is arranged in the region of the main outlet opening (10d) of the nosepiece (10) for the main aerosol flow.

8. Aerosol treatment device according to claim 7, **characterised in that** the nosepiece (10) is formed at one end (10a) for attachment to a connection piece (8, 108) of the nebuliser device (1, 100) and at the other end (10b) for introduction into a naris and the tight closing of the one naris of a user.

9. Aerosol treatment device according to claim 8, **characterised in that** the end (10b) of the nosepiece (10) designed for introduction into a naris is designed in the form of a frustum, preferably with an aperture angle a in the range from 10° to 40°.

10. Aerosol treatment device according to one of the preceding claims, **characterised in that** the flow resistance device (11) is connected to the nosepiece (10) by a connecting element (13).

11. Aerosol treatment device according to one of the preceding claims, **characterised in that** the frequency of the pressure variations lie in the range from 10 to 100 Hz, preferably in the range from 15 to 55 Hz.

12. Aerosol treatment device according to one of the preceding claims, **characterised in that** the pressure variations are generated by means of a membrane compressor, which has a membrane (21) which closes a pressure chamber (20) in pressure-tight manner and which is moved back and forth by a piston rod.

13. Aerosol treatment device according to one of the preceding claims, **characterised in that** the aerosol generator is a nebuliser nozzle (2) with a compressed air channel (5) which leads into a nozzle opening (6), and having at least one intake channel (7), through which a liquid to be nebulised is drawn in, wherein the compressed gas supplied to the nebuliser device also effects the generation of the aerosol.

14. Aerosol treatment device according to one of the preceding claims 1 to 12, **characterised in that** the aerosol generator is a membrane aerosol generator (101) and the nebuliser device (100) furthermore has a supply device (104, 104a, 104b) for supplying the compressed gas (M) for the generation of a main aerosol flow.

15. Aerosol treatment device according to claim 14, **characterised in that** the nebuliser device (100) comprises a cylindrical pipe piece (105) which is arranged around the expansion chamber region (102), in which the aerosol generated by the aerosol generator is expanded such that the supplied compressed gas (M) meets the outer shell surface (105a) of the pipe piece (105) and flows into the interior of the pipe piece (105) at an end-face side end (105b).

16. Aerosol treatment device according to claim 15, **characterised in that** the cylindrical pipe piece (105) is arranged in a chamber (106) surrounded by the nebuliser device such that a gap (107) for the expansion of the supplied compressed gas (M) is formed between an outer shell surface (105a) of the cylindrical pipe piece (105) and an inner wall surface (106a) of the chamber (106).

17. Aerosol treatment device according to claim 15 or 16, **characterised in that** the end-face side end (105b) of the cylindrical pipe piece (105) is arranged in front of the aerosol generator (101).

18. Aerosol treatment device according to one of claims 15 to 17, **characterised in that** the cylindrical pipe piece (105) is the shape of a circular cylinder.

19. Aerosol treatment device according to one of claims 14 to 18, **characterised in that** the supply device for the compressed gas (M) is a cylindrical connection piece (104).

20. Aerosol treatment device according to claim 19, **characterised in that** the cylindrical connection piece (104) has an outlet opening (104a) directed to the cylindrical pipe piece (105).

21. Aerosol treatment device according to claim 20, **characterised in that** the supply device (104) for the compressed gas (M) has a connection device (104b) for connection of a supply pipe for the compressed gas (M).

22. Aerosol treatment device according to one of the preceding claims 1 to 21, **characterised in that** the supply of compressed gas is effected only in predetermined periods of time.

23. Aerosol treatment device according to one of the preceding claims 1 to 22, **characterised in that** an additional compressed gas flow, which is less than the compressed gas flow for the generation of the main aerosol flow, is added to the supplied pressure variations.

## Revendications

1. Système d'aérosolthérapie, comportant
a) un dispositif de nébulisation (1, 100) pouvant être alimenté par un gaz sous pression, préférentiellement par de l'air comprimé, avec un générateur d'aérosol (2, 102) destiné à générer un aérosol et formant un flux principal d'aérosol avec le gaz sous pression alimenté,
b) une pièce nasale (10) raccordée au dispositif de nébulisation (1, 100) pour le refoulement de l'aérosol vers une des deux narines d'un utilisateur,
c) un dispositif de résistance au flux (11) pour l'obtention d'une résistance au flux dans l'autre narine de l'utilisateur, et
d) un dispositif de connexion (10c, 11b) pour l'application de fluctuations de pression superposées au flux principal d'aérosol, ledit dispositif de connexion (10c, 11b) étant formé sur la pièce nasale (10) ou le dispositif de résistance au flux (11), de telle manière que les fluctuations de pression soient directement appliquées sur la narine concernée des deux narines de l'utilisateur.

2. Système d'aérosolthérapie selon la revendication 1, **caractérisé en ce que** le dispositif de résistance au flux (11) comprend le dispositif de connexion (11b) pour l'application des fluctuations de pression, lequel s'étend dans un compartiment intérieur du dispositif de résistance au flux, et présente en outre un premier orifice (11a) pour la communication entre le compartiment intérieur et la cavité nasale de l'utilisateur.

3. Système d'aérosolthérapie selon la revendication 1 ou la revendication 2, **caractérisé en ce que** le dispositif de résistance au flux (11) présente un deuxième orifice (11c), réalisé de telle manière que la résistance au flux est supérieure à la résistance au flux de la voie d'écoulement naturelle par les cavités nasales de l'utilisateur.

4. Système d'aérosolthérapie selon la revendication 2, **caractérisé en ce que** le deuxième orifice (11c) est réalisé dans une paroi du dispositif de résistance au flux (11), laquelle délimite le compartiment intérieur du dispositif de résistance au flux (11).

5. Système d'aérosolthérapie selon l'une des revendications précédentes, **caractérisé en ce que** le dispositif de résistance au flux (11) est un bouchon à introduire dans la cavité nasale.

6. Système d'aérosolthérapie selon la revendication 5, **caractérisé en ce que** le bouchon (11) est réalisé sous la forme d'un cône tronqué, préférentiellement avec un angle d'orifice compris entre 10 et 40°.

7. Système d'aérosolthérapie selon la revendication 1, **caractérisé en ce que** la pièce nasale (10) comporte un dispositif de connexion (10c) pour l'application de fluctuations de pression, ledit dispositif de connexion présentant un orifice de sortie (10e) disposé au niveau de l'orifice de sortie principal (10d) de la pièce nasale (10) pour le flux principal d'aérosol.

8. Système d'aérosolthérapie selon la revendication 7, **caractérisé en ce que** la pièce nasale (10) est prévue à une extrémité (10a) pour la fixation sur une tubulure de raccord (8, 108) du dispositif de nébulisation (1, 100), et à l'autre extrémité (10b) pour l'introduction dans la narine et pour l'obturation étanche de la première narine d'un utilisateur.

9. Système d'aérosolthérapie selon la revendication 8, **caractérisé en ce que** l'extrémité (10b) de la pièce nasale (10) prévue pour l'introduction dans une narine est réalisée avec une forme tronconique, préférentiellement avec un angle d'orifice compris entre 10° et 40°.

10. Système d'aérosolthérapie selon l'une des revendications précédentes, **caractérisé en ce que** le dispositif de résistance au flux (11) est relié par un élément de liaison (13) à la pièce nasale (10).

11. Système d'aérosolthérapie selon l'une des revendications précédentes, **caractérisé en ce que** la fréquence des fluctuations de pression est comprise entre 10 et 100 Hz, préférentiellement entre 15 et 55 Hz.

12. Système d'aérosolthérapie selon l'une des revendications précédentes, **caractérisé en ce que** les fluctuations de pression sont générées au moyen d'un compresseur à membrane, comportant une membrane (21) obturant une chambre de pression (20) de manière étanche à la pression et déplacée en va-et-vient par une tige de piston.

13. Système d'aérosolthérapie selon l'une des revendications précédentes, **caractérisé en ce que** le générateur d'aérosol est une buse de nébulisation (2) avec un canal à air comprimé (5) qui débouche dans un orifice de buse (6), et avec au moins un canal d'aspiration (7) par lequel un liquide à nébuliser est aspiré, le gaz sous pression alimentant le dispositif de nébulisation provoquant également la génération de l'aérosol.

14. Système d'aérosolthérapie selon l'une des revendications 1 à 12, **caractérisé en ce que** le générateur d'aérosol est un générateur d'aérosol à membrane (101), et **en ce que** le dispositif de nébulisation (100) comprend en outre un dispositif d'amenée (104, 104a, 104b) pour l'amenée du gaz sous pression (M) afin de générer un flux principal d'aérosol.

15. Système d'aérosolthérapie selon la revendication 14, **caractérisé en ce que** le dispositif de nébulisation (100) comprend une pièce de conduite cylindrique (105) disposée autour de la zone de chambre d'expansion (102) où se développe l'aérosol généré par le générateur d'aérosol, de telle manière que le gaz sous pression (M) amené rencontre la surface latérale extérieure (105a) de la pièce de conduite (105) et s'écoule à l'intérieur de la pièce de conduite (105) par une extrémité frontale (105b).

16. Système d'aérosolthérapie selon la revendication 15, **caractérisé en ce que** la pièce de conduite cylindrique (105) est disposée dans une chambre (106) entourée par le dispositif de nébulisation, de manière à former un interstice (107) pour l'expansion du gaz sous pression (M) amené entre une surface latérale extérieure (105a) de la pièce de conduite (105) et une surface intérieure de paroi (106a) de la chambre (106).

17. Système d'aérosol thérapie selon la revendication 15 ou la revendication 16, **caractérisé en ce que** l'extrémité frontale (105b) de la pièce de conduite cylindrique (105) est disposée devant le générateur d'aérosol (101).

18. Système d'aérosolthérapie selon l'une des revendications 15 à 17, **caractérisé en ce que** la pièce de conduite cylindrique (105) est en forme de cylindre circulaire.

19. Système d'aérosolthérapie selon l'une des revendications 14 à 18, **caractérisé en ce que** le dispositif d'amenée du gaz sous pression (M) est une tubulure de raccord cylindrique (104).

20. Système d'aérosolthérapie selon la revendication 19, **caractérisé en ce que** la tubulure de raccord cylindrique (104) présente un orifice de sortie (104a) opposé à la pièce de conduite cylindrique (105).

21. Système d'aérosolthérapie selon la revendication 20, **caractérisé en ce que** le dispositif d'amenée (104) du gaz sous pression (M) comporte un dispositif de connexion (104b) pour le raccordement à une conduite d'amenée du gaz sous pression (M).

22. Système d'aérosolthérapie selon l'une des revendications 1 à 21, **caractérisé en ce que** l'amenée du gaz sous pression n'est effectuée qu'à intervalles temporels définis.

23. Système d'aérosolthérapie selon l'une des revendications 1 à 22, **caractérisé en ce qu'**un flux additionnel de gaz sous pression est ajouté aux fluctuations de pression appliquées, lequel est inférieur au flux de gaz sous pression pour la génération du flux principal d'aérosol.
